# EUROPEAN PATENT APPLICATION

(11) **EP 1 632 226 A1**
(43) Date of publication of application: **08.03.2006**
(21) Application number: 04745727.0
(22) Date of filing: 10.06.2004
(51) Int. Cl.: A61K 31/245, A61K 31/125, A61K 9/70, A61K 31/60, A61K 45/00, A61P 29/00

(54) **ANTI-INFLAMMATORY ANALGESIC ADHESIVE PATCH**

(30) Priority: 11.06.2003 JP 2003166774
(71) Applicant: TEIKOKU SEIYAKU CO., LTD., Higashikagawa-shi Kagawa 769-2695 (JP)
(72) Inventor: KONISHI, Tatsuya, Kagawa 7692601 (JP); ABE, Yasuko, Sanuki-shi, Kagawa 7692313 (JP); MACHIDA, Yuji, Kagawa 7692704 (JP)
(74) Representative: Albrecht, Thomas
(86) International application number: PCT/JP2004/008097
(87) International publication number: WO 2004/110428

(57) **Abstract**

An anti-inflammatory analgesic adhesive patch which has an excellent anti-inflammatory analgesic effect and is reduced in unpleasant irritant feeling during wear. It is characterized by containing, as active ingredients, benzocaine and an ingredient having a counter-irritation effect, wherein the ingredient having a counter-irritation effect comprises one or more members selected from the group consisting of 1-menthol, dl-menthol, dl-camphor, d-camphor, methyl salicylate, glycol salicylate, peppermint oil, eucalyptus oil, capsaicin, capsicum extract, and nonylic acid vanillamide and the benzocaine is contained in an amount of 0.5 to 20 wt.%

## Description

### TECHNICAL FIELD

The present invention relates to a patch containing, as active ingredients, benzocaine and an ingredient having a counter-irritation effect, for use with muscle pain, joint pain, lumbago, shoulder stiffness, fracture pain, and other symptoms associated with pain. More particularly, the present invention relates to an analgesic and anti-inflammatory patch that reduces an unpleasant irritation when applied, while being highly effective.

### TECHNICAL BACKGROUND

Many analgesic and anti-inflammatory patches are sold that comprise the following elements: a backing, such as nonwoven fabric, woven fabric, and polyvinyl chloride film; a drug-containing adhesive layer deposited on the backing and containing active ingredients, such as 1-menthol, dl-camphor, and methyl salicylate; and a peelable film, such as polypropylene film, polyethylene terephthalate film, and paper, laminated on the drug-containing adhesive layer.

Many patches are also sold that contain, in addition to the above-described active ingredients, calefacients such as capsicum extracts and nonylic vanillylamide and are widely used with lumbago, shoulder stiffness and other chronic symptoms to cause warm sensation.

The components such as 1-menthol, dl-camphor, methyl salicylate, capsicum extracts, and nonylic vanillylamide that are added to the patches are intended to act as "counter-irritants."

Ingredients having a counter-irritation effect, so-called counter-irritants, are agents that cause a slight inflammation upon a topical application to the skin and thereby dissipate the congestion in the tissue below. Counter-irritants are used to alleviate the congestion in the deep tissue by taking advantage of their ability to stimulate the skin and cause a slight inflammation.

However, the counter-irritants such as 1-menthol and methyl salicylate often cause pain and rash where the patch is applied depending on the type of symptoms. In addition, the calefacients used in the patches elicit different degrees of heat sensation depending on age, sex, and an application site. In some cases, the results are an unpleasant skin irritation such as rubor and rash. The irritation may persist after removal of the patch and may sometimes be experienced as a strong pain during, for example, bathing.

To reduce such an irritation and a pain, various natural medicines have been used in the patches, but none have proven sufficiently effective and a need exists for improved products.

### DISCLOSURE OF THE INVENTION

### PROBLEMS TO BE SOLVED BY THE INVENTION

In view of the present state of the art, it is an objective of the present invention to provide an analgesic and anti-inflammatory patch that has a high anti-inflammatory and analgesic effect and at the same time causes reduced an unpleasant irritation upon an application.

In an effort to achieve the foregoing objective, the present inventors have devoted efforts to seeking a measure to alleviate a skin irritation and, as a result, have found that a patch that contains benzocaine as an active ingredient, along with an effective amount of a counter-irritant, reduces an unpleasant irritation, while having a high anti-inflammatory and analgesic effect. It is this discovery that led to the present invention.

### MEANS FOR SOLVING THE PROBLEMS

Accordingly, one essential aspect of the present invention is an analgesic and anti-inflammatory patch containing, as active ingredients, benzocaine and an ingredient having a counter-irritation effect.

Specifically, the analgesic and anti-inflammatory patch of the preset invention contains benzocaine in an effective amount of 0.5 to 20 wt%.

More specifically, the analgesic and anti-inflammatory patch of the present invention contains, along with benzocaine, at least one ingredient having a counter-irritation effect selected from the group consisting of 1-menthol, dl-menthol, dl-camphor, d-camphor, methyl salicylate, glycol salicylate, mentha oil, eucalyptus oil, capsaicin, capsicum extract, and nonylic vanillylamide.

Thus, the analgesic and anti-inflammatory patch of the present invention is characterized in that it contains effective amounts of benzocaine and an ingredient having a counter-irritation effect.

More specifically, the analgesic and anti-inflammatory patch of the present invention contains the ingredient having a counter-irritation effect in an amount of 0.01 to 30 wt% when it is one of 1-menthol, dl-menthol, dl-camphor, d-camphor, methyl salicylate, glycol salicylate, mentha oil and eucalyptus oil, or in an amount of 0.001 to 5 wt% when it is capsaicin, one of capsicum extract and nonylic vanillylamide.

In a specific embodiment of the analgesic and anti-inflammatory patch of the present invention, the patch is provided in the form of an aqueous poultice containing 10 to 80 wt% water. More specifically, the analgesic and anti-inflammatory patch comprises the aqueous poultice material containing 10 to 80 wt% water, 0.5 to 20 wt% of benzocaine, and at least one ingredient having a counter-irritation effect selected from the group consisting of 1-menthol, dl-menthol, dl-camphor, d-camphor, methyl salicylate, glycol salicylate, mentha oil, eucalyptus oil, capsaicin, capsicum extract, and nonylic vanillylamide.

Another embodiment of the present invention concerns the use of benzocaine in the reduction of the skin irritation caused by an ingredient having a counter-irritation effect used in the analgesic and anti-inflammatory patch as an active ingredient.

### ADVANTAGE OF THE INVENTION

Thus, the present invention provides an analgesic and anti-inflammatory patch that has a high anti-inflammatory and analgesic effect while reducing an unpleasant irritation upon an application. The patch of the present invention contains an ingredient having a counter-irritation effect, along with benzocaine, as active ingredients, so that it causes less skin irritation than the conventional patch preparations containing counter-irritants while exhibiting a desired anti-inflammatory and analgesic effect.

### BEST MODE FOR CARRYING OUT THE INVENTION

The present invention will now be described in detail.
The analgesic and anti-inflammatory patch of the present invention, which contains as active ingredients benzocaine in combination with an ingredient having a counter-irritation effect, reduces a skin irritation upon an application while exhibiting a high anti-inflammatory and analgesic effect.

To make such an analgesic and anti-inflammatory patch, the amount of benzocaine added is preferably 0.5 to 20 wt%, more preferably 5 to 15 wt%, of the paste. Benzocaine added in amounts of less than 0. 5 wt% cannot provide a sufficient anti-inflammatory and analgesic effect, while the compound when added in amounts of more than 20 wt% reduces the stability of the resulting preparation.

The ingredients having a counter-irritation effect added to the patch of the present invention along with benzocaine include 1-menthol, d1-menthol, dl-camphor, d-camphor, methyl salicylate, glycol salicylate, mentha oil, eucalyptus oil, capsaicin, capsicum extract, and nonylic vanillylamide. These counter-irritants may be used either individually or in combination.

The ingredient having a counter-irritation effect may be added in any amount commonly used in analgesic and anti-inflammatory patches. Specifically, the ingredient such as 1-menthol, dl-menthol, dl-camphor, d-camphor, methyl salicylate, glycol salicylate, mentha oil and eucalyptus oil is added preferably in an amount of 0.01 to 30 wt%, and more preferably in an amount of 0. 1 to 15 wt% of the preparation. The ingredient such as capsaicin, capsicum extract, and nonylic vanillylamide is added preferably in an amount of 0.001 to 5 wt%, and more preferably in an amount of 0.005 to 3 wt% of the preparation.

The counter-irritants added in amounts less than the specified range cannot achieve desired counter-irritant effects in the resulting patches, while the skin irritation of the resulting patches become unfavorably strong when the counter-irritants are added in amounts greater than the specified range.

Aside from the above-described components, the analgesic and anti-inflammatory patch provided in accordance with the present invention may contain other components commonly used in external preparations. Among such components are tocopherol acetate and other vitamin E family members, nicotinamide and benzyl nicotinate, each of which facilitates a blood circulation; glycyrrhetin, glycyrrhizinic acid and dipotassium glycyrrhizinate, each of which has an anti-inflammatory and analgesic effect; diphenhydramine and chlorpheniramine maleate,each having an anti-allergic effect;plant extracts, such as arnica tincture, phellodendron bark extract, chamomile extract, gardenia fruit extract, zanthoxylum fruit extract, lithospermum root extract, aesculus hippocastanum seed extract, and swertia herb extract; preservatives, including parabens, such as methyl parahydroxybenzoate, ethyl parahydroxybenzoate, propyl parahydroxybenzoate, and butyl parahydroxybenzoate, phenoxyethanol, benzoic acid, salicylic acid,benzethonium chloride, cetylpyridinium chloride, and chlorhexidine hydrochloride; and antioxidants, such as butylhydroxyanisol, dibutylhydroxytoluene, ascorbic acid, and sodium ascorbate.

The analgesic and anti-inflammatory patch of the present invention may be provided in the form of a poultice, a plaster, a tape or any other suitable preparations. The base for use in these preparations may be any base commonly used in patches.

When the analgesic and anti-inflammatory patch of the present invention is provided in the form of an aqueous poultice, the base used is preferably awater-soluble polymer, such as gelatin, pullulan, polyvinylpyrrolidone, polyvinylalcohol, methylcellulose, ethylcellulose, carboxymethylcellulose, hydroxyethylcellulose, hydroxypropylcellulose, polyacrylic acid, sodium polyacrylate, acrylic acid copolymer, maleic anhydride copolymer, starch/sodium acrylate graft copolymer, carboxyvinyl polymer, sodium alginate, xanthan gum, agar, and carrageenan. These polymers may be used either individually or in combination and are typically added in amounts of 3 to 40 wt% relative to the total weight of the paste, while the amount may vary depending on the desired strength of base and cooling capacity of the patch and the desired handleability of the material during production.

When it is desired to crosslink the water-soluble polymer to serve as the base component of the aqueous poultice, a crosslinker is used, including polyvalent metals, such as potassium alum, aluminum alum, magnesium chloride, calcium chloride, aluminum chloride, aluminum hydroxide, calcium hydroxide, ferric hydroxide, calcium phosphate, calcium citrate, aluminum glycinate, magnesium aluminometasilicate, magnesium aluminosilicate, aluminum metasilicate, magnesium silicate, and synthetic hydrotalcite, and polyethyleneglycol diglycidyl ether, ethyleneglycol diglycidyl ether, glycerin diglycidyl ether, and triglycerin diglycidyl ether. These crosslinkers are added preferably in an amount of 0.001 to 5 wt%, and more preferably in an amount of 0.005 to 3 wt% relative to the total weight of the paste. The crosslinkers may be used either individually or in combination.

The crosslinker is preferably used in conjunction with a crosslinking adjustor, including organic acids, such as citric acid, malic acid, tartaric acid, glycolic acid, fumaric acid, succinic acid and edetic acid, and salts thereof. The crosslinking adjustors may be used either individually or in combination.

The base may further contain an inorganic salt, such as kaolin, bentonite, and titanium oxide; and a commonly used absorption enhancers, such as castor oil, crotamiton, isopropyl myristate, isopropyl adipate, diisopropyl sebacate, diisopropanolamine and N,N-diethyl-m-toluamide. These components are added typically in an amount of 0.01 to 20 wt%, and preferably in an amount of 0.1 to 10 wt% relative to the weight of the paste. The base may also contain a polyhydric alcohol, such as ethylene glycol, diethylene glycol, polyethylene glycol, propylene glycol, polypropylene glycol, 1,3-butylene glycol, glycerol, and sorbitol. The polyhydric alcohols are added preferably in an amount of 3 to 60 wt%, and preferably in an amount of 10 to 50 wt% relative to the total weight of the paste.

The aqueous poultice to serve as one embodiment of the analgesic and anti-inflammatory patch of the present invention preferably contains 10 to 80 wt% water, and more preferably 30 to 75 wt% water, relative to the total weight of the paste.

The pH of the aqueous poultice material is adjusted typically to a range of 3.0 to 9.0, preferably to a range of 3.5 to 8.0, and more preferably to a range of 4.0 to 7.5. The poultice having a pH lower than 3 . 0 is excessively acidic and thus causes a significant skin irritation, whereas the material with a pH higher than 9.0 is corrosive and damages the skin.

The backing for use in the aqueous poultice may be a nonwoven fabric, a woven fabric, or a laminate of a nonwoven or woven fabric with polyethylene, polypropylene, polyvinyl chloride, polyvinylidene chloride, polyurethane or polyethylene terephthalate. Of these materials, a nonwoven fabric is particularly preferred. Examples of preferred nonwoven fabrics are those made of at least one fiber selected from chemical fibers, such as nylon, vinylidene, polyvinyl chloride, polyester, acryl, polyethylene, polypropylene, and polyurethane, and natural fibers, such as cotton, wool, hemp, and silk.

In another embodiment, the analgesic and anti-inflammatory patch of the present invention may be provided in the form of a plaster. In such cases, the adhesive for use with the plaster may be a synthetic rubber adhesive such as styrene-isoprene-styrene block copolymer, or it may be a natural rubber adhesive, hydrogenated petroleum resin, rosin, hydrogenated rosin, polyvinyl alcohol, or polyvinyl pyrrolidone. The amount of the adhesive is preferably 15 to 80 wt% of the total weight of the paste.

The plaster may contain a softener, such as liquid rubber including polybutene and polyisobutylene, liquid paraffin, vegetable oil, and lanolin. The softeners are preferably added in an amount of 10 to 40 wt% relative to the total weight of the paste. If necessary, the plaster may further contain an agent for facilitating a drug transdermal absorption such as fatty acid esters and higher alcohols and the above-described components commonly used in external preparations.

The backing for use in the plaster may be a resin film, such as cellulose derivativefilm,polyethylene terephthalatefilm,nylon film, polyvinyl chloride film, polyethylene film, polyurethane film, and polyvinylidene chloride film, a metal sheet, such as aluminum sheet, a nonwoven fabric, or a woven fabric. The resin films and metal sheets may be used individually or they may be laminated to, for example, a nonwoven fabric.

When provided in the form of aqueous poultice, the analgesic and anti-inflammatory patch of the present invention can be manufactured as follows: Benzocaine, an ingredient having a counter-irritation effect, and other optional components are mixed together to form, for example, a paste. The paste is applied to paper, a woven fabric, a nonwoven fabric, a plastic film or other backings (backing materials) to a predetermined thickness. A clear protective film, such as polyethylene film, is then laminated to the paste coating and the laminate is cut into pieces of desired size. While the drug-containing pastemaybe applied to the substrate sheet to form a layer of any weight, the amount of coating is typically 200 to 2000g/m², and preferably 400 to 1500g/m².

When provided in the form of plaster, the analgesic and anti-inflammatory patch can be manufactured as follows: a synthetic rubber adhesive such as styrene-isoprene-styrene block copolymer, a softener, a tackifier, an antioxidant, and a filler are melted, mixed and kneaded together. To this mixture, benzocaine, an ingredient having a counter-irritation effect, and other optional components are added and the mixture is kneaded until uniform. The kneaded product is spread over a liner and the film is cut into pieces of desired size and shape.

### Examples

The analgesic and anti-inflammatory patch of the present invention will now be described in specific detail with reference to examples and comparative examples, which are not intended to limit the scope of the invention in any way.

### Examples 1 through 3: Aqueous poultice

Three different aqueous poultice bases having the compositions shown in Table 1 below were prepared using a conventional technique. Each base was applied to a piece of nonwoven fabric to a uniform thickness, and a polyethylene terephthalate film was laminated to the nonwoven fabric over the base. This gave aqueous poultices of Examples 1 through 3.

**[Table 1]**

| Components | Amount (wt%) | | |
|---|---|---|---|
| | Example 1 | Example 2 | Example 3 |
| Methyl salicylate | 5.0 | - | 1.0 |
| 1-menthol | - | 1.0 | - |
| dl-camphor | - | 1.0 | 1.0 |
| Capsicum extract | - | - | 0.3 |
| Benzocaine | 5.0 | 5.0 | 5.0 |
| Castor oil | 0.5 | 1.0 | 0.5 |
| Aluminum hydroxide | 0.01 | 0.01 | 0.01 |
| Magnesium aluminometasilicate | 0.03 | 0.03 | 0.03 |
| Partially neutralized polyacrylic acid | 5.0 | 5.0 | 5.0 |
| Polyacrylic acid | 4.0 | 4.0 | 4.0 |
| Carmellose sodium | 3.0 | 3.0 | 3.0 |
| Glycerol | 16.0 | 16.0 | 16.0 |
| Tartaric acid | 0.5 | 0.5 | 0.5 |
| N,N-diethyl-m-toluamide | 6.0 | 6.0 | 6.0 |
| Disodium edetate | 0.04 | 0.04 | 0.04 |
| Methyl parahydroxybenzoate | 0.15 | - | - |
| Butylhydroxyanisol | - | 0.2 | - |
| Butylhydroxytoluene | - | 0.2 | - |
| Purified water | 54.77 | 57.02 | 57.62 |

### Comparative Examples 1 through 3:

Three different benzocaine-free aqueous poultice bases having the compositions shown in Table 2 below were prepared using a conventional technique. Each base was applied to a piece of nonwoven fabric to a uniform thickness, and a polyethylene terephthalate film was laminated to the nonwoven fabric over the base. This gave aqueous poultices of Comparative Examples 1 through 3.

**[Table 2]**

| Components | Amount (wt%) | | |
|---|---|---|---|
| | Comparative example 1 | Comparative example 2 | Comparative example 3 |
| Methyl salicylate | 5.0 | - | 1.0 |
| 1-menthol | - | 1.0 | - |
| dl-camphor | - | 1.0 | 1.0 |
| Capsicum extract | - | - | 0.3 |
| Castor oil | 0.5 | 1.0 | 0.5 |
| Aluminum hydroxide | 0.01 | 0.01 | 0.01 |
| Magnesium aluminometasilicate | 0.03 | 0.04 | 0.04 |
| Partially neutralized polyacrylic acid | 5.0 | 5.0 | 5.0 |
| Polyacrylic acid | 4.0 | 4.0 | 4.0 |
| Carmellose sodium | 3.0 | 3.0 | 3.0 |
| Glycerol | 16.0 | 16.0 | 16.0 |
| Tartaric acid | 0.5 | 0.5 | 0.5 |
| N,N-diethyl-m-toluamide | 6.0 | - | - |
| Disodium edetate | 0.04 | 0.04 | 0.04 |
| Methyl parahydroxybenzoate | 0.15 | - | - |
| Butylhydroxyanisol | - | 0.2 | - |
| Butylhydroxytoluene | - | 0.2 | - |
| Purified water | 59.77 | 68.01 | 68.61 |

### Example 4: Plaster

According to the formula below, a styrene-isoprene-styrene block copolymer, an alicyclic saturated hydrocarbon resin, a synthetic rubber, and dibutylhydroxytoluene were melted and kneaded together. To the kneaded product, nonylic vanillylamide, methyl salicylate, dl-camphor, and benzocaine were added and the material was further kneaded until uniform. The product was then spread over a liner to obtain a plaster.

**Formula:**

| | |
|---|---|
| nonylic vanillylamide | 0.01 wt% |
| methyl salicylate | 5.0 wt% |
| dl-camphor | 2.0 wt% |
| benzocaine | 5.0 wt% |
| styrene-isoprene-styrene block copolymer | 22.0 wt% |
| alicyclic saturated hydrocarbon resin | 46.99 wt% |
| synthetic rubber | 18.0 wt% |
| dibutylhydroxytoluene | 1.0 wt% |

### Test Example 1:

The aqueous poultices of Examples 1 through 3 and Comparative Examples of 1 through 3 and the plaster of Example 4 were each applied to 20 human subjects on the inner surface of the brachium. The degree of an irritation felt by the subjects was evaluated by the sensitivity test 10, 20, 30, 60, and 90 minutes after an application.
The irritation was rated on a scale of 0 to 4, where:
4 = strong pain;
3 = irritation accompanied by pain;
2 = moderate irritation;
1 = weak irritation; and
0 = no irritation.

An average was taken of 10 subjects. The results are shown in Table 3.

**[Table 3]**

| | 10 min. | 20 min. | 30 min. | 60 min. | 90 min. |
|---|---|---|---|---|---|
| Example 1 | 1.2 | 1.9 | 2.2 | 2.2 | 2.2 |
| Example 2 | 1.1 | 1.8 | 2.1 | 2.2 | 2.1 |
| Example 3 | 1.1 | 1.9 | 2.2 | 2.1 | 2.2 |
| Example 4 | 0.9 | 1.7 | 2.3 | 2.2 | 2.2 |
| Comparative Example 1 | 1.1 | 2.5 | 3.1 | 3.2 | 3.2 |
| Comparative Example 2 | 1.2 | 2.4 | 2.6 | 2.6 | 2.4 |
| Comparative Example 3 | 1.3 | 1.8 | 2.6 | 2.7 | 2.7 |

As can be seen from the results of Table 3, each of the patches of the present invention (Examples 1 through 4) caused a moderate irritation approximately 20 minutes after the application of the patches. During the time period of 30 to 90 minutes, the subjects applied any of the patches of the invention felt only a mild irritation and claimed less pain than those who were applied the patches of Comparative Examples.

### Test Example 2:

The patches of Example 1 and Comparative Example 1 were applied to 10 volunteered subjects suffering from joint pain (informed consent was obtained under the supervision of medical doctors) and the effect was evaluated.
The effect was rated based on the following criteria:
++ = pain was significantly decreased;
+ = pain was slightly decreased;
+- = no change; and
- = pain increased.

The results are shown in Table 4.

**[Table 4)**

| Criteria | Patch of Example 1 | Patch of Comparative Example 1 |
|---|---|---|
| ++ | 5 | 3 |
| + | 2 | 2 |
| +- | 3 | 5 |
| - | 0 | 0 |

### Test Example 3:

The patches of Example 3 and Comparative Example 3 were applied to 10 volunteered subjects suffering from lumbago (informed consent was obtained under the supervision of medical doctors) and the effect was evaluated.
The evaluation criteria were as follows:
++ = pain was significantly decreased;
+ = pain was slightly decreased;
+- = no change; and
- = pain increased.

The results were shown in Table 5.

**[Table 5]**

| Criteria | Patch of Example 3 | Patch of Comparative example 3 |
|---|---|---|
| ++ | 4 | 4 |
| + | 4 | 2 |
| +- | 2 | 4 |
| - | 0 | 0 |

As demonstrated by the results of Tables 4 and 5, the patches of the present invention turned out to be effective against joint pain and lumbago in comparison with the comparative examples.

### INDUSTRIAL APPLICABILITY

As set forth, the patch of the present invention, which contains, as active ingredients, benzocaine and an ingredient having a counter-irritation effect, causes reduced skin irritation in comparison with the conventional preparation containing an ingredient having a counter-irritation effect, while exhibiting desired anti-inflammatory and analgesic effects. It is thus of significant medical importance.

## Claims

1. An analgesic and anti-inflammatory patch comprising, as active ingredients, benzocaine and an ingredient having a counter-irritation effect.

2. The analgesic and anti-inflammatory patch according to claim 1, containing benzocaine in an amount of 0.5 to 20 wt%.

3. The analgesic and anti-inflammatory patch according to claim 1 or 2, wherein the ingredient having a counter-irritation effect is at least one selected from the group consisting of 1-menthol, dl-menthol, dl-camphor, d-camphor, methyl salicylate, glycol salicylate, mentha oil, eucalyptus oil, capsaicin, capsicum extract, and nonylic vanillylamide.

4. The analgesic and anti-inflammatorypatch according to claim 3, containing the ingredient having a counter-irritation effect in an amount of 0.01 to 30 wt% when it is one of 1-menthol, dl-menthol, dl-camphor, d-camphor, methyl salicylate, glycol salicylate, mentha oil and eucalyptus oil, or in an amount of 0.001 to 5 wt% when it is capsaicin, one of capsicum extract and nonylic vanillylamide.

5. The analgesic and anti-inflammatory patch according to any of claims 1 to 4, provided in the form of an aqueous poultice containing 10 to 80 wt% water.

6. An analgesic and anti-inflammatory patch comprising an aqueous poultice material containing 10 to 80 wt% water, 0.5 to 20 wt% of benzocaine, and at least one ingredient having a counter-irritation effect selected from the group consisting of 1-menthol, dl-menthol, dl-camphor, d-camphor, methyl salicylate, glycol salicylate, mentha oil, eucalyptus oil, capsaicin, capsicum extract, and nonylic vanillylamide.

7. Use of benzocaine in the reduction of the skin irritation caused by an ingredient having a counter-irritation effect used in an analgesic and anti-inflammatory patch as an active ingredient.
